# Europäisches Patentamt

## European Patent Office

## Office européen des brevets

(19)

(11) Publication number: **0 041 692**
**B2**

(12)

# NEW EUROPEAN PATENT SPECIFICATION

(45) Date of publication of the new patent specification:
29.03.89

(51) Int. Cl.⁴: **A 61 M 1/18**, A 61 M 1/20, A 61 M 1/22

(21) Application number: **81104274.6**

(22) Date of filing: **03.06.81**

(54) Blood oxygenator.

(30) Priority: 06.06.80 JP 76816/80

(43) Date of publication of application:
16.12.81 Bulletin 81/50

(45) Publication of the grant of the patent:
09.05.84 Bulletin 84/19

(45) Mention of the opposition decision:
29.03.89 Bulletin 89/13

(84) Designated Contracting States:
DE FR GB IT

(56) References cited:
DE-A-2 639 549
DE-B-1 794 191
GB-A-2 010 695
GB-A-2 014 060
US-A-3 733 776
US-A-3 894 954
US-A-3 993 566
US-A-4 020 230
US-A-4 124 510

"Development of Polyalkylsulfone Membranes for
Blood Oxygenators" A. D. Little, PB-257438, U.S.
Department of Commerce NTIS
"Use of a Capillary Membrane Oxygenator for Total
Cardiopulmonary Bypass in Calves" M.P. Kaye et al
Surgical Research 14(1973) pages 58-63
Polymer Handbook, 2nd Edition, Brandrup and
Immergut, pages III-221 to III-227
"Polyalkylsulfone: A new polymer for membrane

(73) Proprietor: NIKKISO CO., LTD., No. 43- 2, Ebisu
3-chome, Shibuya- ku Tokyo (JP)
Proprietor: KANEGAFUCHI KAGAKU KOGYO
KABUSHIKI KAISHA, 2-4 Nakanoshima 3-chome,
Kita- ku Osaka- shi Osaka- fu (JP)

(72) Inventor: Sumoto, Misao, c/o Nikkiso Mitakaryo
9-5 shimorenjaku 6-Chome, Mitaka- shi Tokyo (JP)
Inventor: Imai, Koichi, 1968- 9 Katsumata
Haibaramachi, Haibara- gun Shizuoka- ken (JP)
Inventor: Kambayashi, Tomio, 130- 1 Setoaraya,
Fujiedashi Shizuoka- ken (JP)
Inventor: Ikeuchi, Tatsuro, 4,Shimoyamatedori
4-Chome Chuo- ku, Kobe (JP)
Inventor: Eguchi, Tamiyuki, 2-8 Yoshidacho
1-Chome Hyogo- ku, Kobe (JP)
Inventor: Kuge, Tadao, 21- 8 Matsubaracho,
Takatsuki- shi Osaka- fu (JP)

(74) Representative: Kinzebach, Werner, Dr.,
Patentanwälte Reitstötter, Kinzebach und
Partner Sternwartstrasse 4 Postfach 86 06 49,
D-8000 München 86 (DE)

(56) References cited: (continuation)
oxygenator" Transactions American Society for
Artificial Internal Organs", Ketteringham et al,
Volume XIX, 1973 pages 61 to 65

EP 0 041 692 B2

LIBER, STOCKHOLM 1989

## Description

This invention relates to a blood oxygenator for oxygenating blood and simultaneously removing carbon dioxide therefrom.

Blood oxygenators have been developed and used in practice as a component for effecting extracorporeal circulation during cardiotomy surgery. The function of blood oxygenator is to transfer oxygen into the venous blood with simultaneous removal of carbon dioxide therefrom.

Three principal types of blood oxygenators are known. In the bubble oxygenator, bubbles of oxygen gas are directly introduced into the blood. The bubble oxygenator is generally disposable after use, easy to handle, reliable in oxygenation performance, and is, therefore, currently in wide use. However, there exist certain defects of remarkable blood damage such as hemolysis and the danger of introducing fine oxygen gas bubbles into the patient resulting in vascular embolism.

In the rotary disc oxygenator, a thin film of blood is exposed to an oxygen atmosphere. Here, hemolysis is less remarkable than in bubble oxygenators but it suffers certain disadvantages such as larger priming volume and difficulty of construction in disposable form.

In the membrane oxygenator, a gas-permeable membrane separates the blood from the oxygen gas, and gas exchange takes place by diffusion across the membrane. Such a membrane which is used in a reverse osmosis apparatus is described in the US-A-3 993 566. This type of oxygenator has less priming volume and less remarkable hemolytic characteristic than other two types, and has been increasingly used in practice.

As the basic requirements, the membrane must have the following properties:

(1) a high permeability to oxygen gas and carbon dioxide gas.
(2) a good bio-compatibility, particularly minimum hemolysis.
(3 no pinholes and a good mechanical strength.

One type of membrane such as a silicone membrane utilizes the dissolution and diffusion of oxygen gas or carbon dioxide gas in the membrane material. Not only in the membrane oxygenator but also in other membrane diffusion devices, it is well-known that the diffusion rate depends on not only that of the membrane itself but also that of the boundary film of fluid formed on the both sides of membrane. Accordingly, the overall resistance to diffusion is the sum of intramembrane resistance plus boundary film resistance. If the former is negligibly low, the mass transfer rate through a given membrane is mainly controlled by the boundary film resistance. It is for this reason that the silicone membrane has relatively high permeabilities to oxygen gas and carbon dioxide gas. The silicone membrane of sufficient thichness exhibits, however, a poor $CO_2$ releasing rate compared with its $O_2$ absorption rate and often causes hemolysis when the membrane is reinforced with fillers such as fine silica particles.

There is another type of membrane which employs a quite different permeation mechanism from that of the silicone membrane. It comprises a hydrophobic microporous membrane such as microporous polypropylene membranes sold by Cellanese Corporation under commercial names of Cellguard(T) and Duraguard(T). These membranes have a number of elongated microholes having a pore size, for example, of $0.02 \times 0.4$ μm and a porosity up to several tens percent. Because of its hydrophobic nature, the membrane prevents the passage of water but permits the passage of a gas through the membrane below a predetermined level of pressure. Thus, the oxygen and carbon dioxide can diffuse in opposite directions through the microholes of the membrane without allowing blood passage through the membrane, so that absorption of oxygen and release of carbon dioxide take place. The gas-permeability of membrane of this type is, therefore, far greater than that of a silicone membrane, and the diffusion constant thereof will be at least 1.000 times higher than that of a silicone membrane even the average pore size is sufficiently small to allow Knudsen flow of gas, namely about 0.01 μm. This enables the reduction of the surface area of the membrane at least by about 20 % compared with the silicone membrane oxygenator, although more drastic reduction of surface area is impossible because the mass transfer rate is predominantly controlled by the boundary film resistance.

Other examples of membranes of this class include microporous polytetrafluoroethylene membranes, microporous polycarbonate membranes, silicone or paraffin treated microporous hydrophilic membranes and the like. All membranes of this class are, however, of an isotropic structure and have an average pore size greater than 0.01 μm.

In the report "Development of Polyalkylsulfone Membranes for Blood Oxygenators", A.D.Little, PB-257 438, US Department of Commerce NTIS, a membrane is described which consists of a porous polypropylene substrate coated with a thin polyalkyl sulfone film.

## Description of the invention

In accordance with the present invention, a blood oxygenator is provided for oxygenating blood and simultaneously removing carbon dioxide therefrom comprising a semi-permeable non-hydrophilic membrane and a first flow path and a second flow path positioned on opposite sides of said membrane in gas exchange relation, whereby the membrane

- is made of polysulfone resin,
- has a critical surface tension less than the surface tension of human blood at 37°C,
- comprises a porous layer having a thickness of 10 to 500 µm and a pore size of 0.1 to 20 µm and integral with the porous layer a skin layer of 0.01 to 3 µm thickness, having minute pores of a pore size of 1 nm to 10 nm.

The skin layer is dry in use and the pressure of blood side is maintained at a level higher than the pressure of oxygen gas side but less than the minimum pressure at which the blood begins to penetrate into the membrane.

The membrane oxygenator of the present invention is more effective than conventional prior art membrane oxygenators. For example, a membrane oxygenator having a membrane surface area of 0.8 m² was assembled using an anisotropic membrane of 50 µm total thickness made of dichlorodiphenylsulfone-bisphenol condensate resin having a porosity of 70 %, and a skin layer of 1 µm thick having an average pore size of less than 0.01 µm. This oxygenator was tested by flowing blood at a pressure of 27.1 kPa on the skin layer side and oxygen gas at a pressure of 266,6 Pa on the porous layer side. At a partial pressure of oxygen gas of 5,33 kPa and a partial pressure of carbon dioxide of 6,66 kPa gas in the blood, the oxygenator exhibited an $O_2$ absorption rate and a $CO_2$ releasing rate which are 1.3 times and 2.2 times, respectively, higher than those of a silicone membrane oxygenator having a corresponding effective surface area of 100 µm thick under same conditions, or 1.2 times and 1.3 times, respectively, higher than those of a porous polypropylene membrane oxygenator of 25 µm thick having a number of elliptic pores of an average size of 0.02 x 0.4 µm and a porosity of 50 % under same conditions.

Thus, the oxygenator of this invention is superior to prior art membrane oxygenators in its high $CO_2$ expulsion rate. This is because the membrane used in this invention is of anisotropic structure having a compact skin layer and a porous layer which is substantially less resistant to gas permeation so that the overall resistance of the membrane to gas permeation is nearly equal to that of the skin layer of, for example, 1 µm thick. The porous layer serves as a reinforcement.

Blood damage such as hemolysis in the oxygenator of this invention is minimum or far less than in silica filled silicone membrane oxygenators and conventional microporous membrane oxygenators. This may be explained by the fact that blood flows while being in contact with a relatively smooth surface of skin layer and the polysulfonate resin used has a high compatibility with blood.

One of the characteristic features of this invention resides in the use of a microporous, anisotropic membrane having a compact skin layer of the above-described type. It is preferable to place the membrane so that its skin layer faces the blood side and the porous layer faces the oxygen gas side within the oxygenator of the present invention. The orientation of membrane in this manner gives additional effects in that the permeability to gas is greater than in reverse orientation and that the danger of passage of oxygen gas bubbles into the blood is minimized even when the pressure of oxygen side becomes higher than the pressure of blood side by an accident.

Another characteristic feature of the oxygenator of this invention is that the membrane is non-hydrophilic and has a critical surface tension less than the surface tension of blood at 37°C. The pressure of blood side separated by the membrane from the oxygen gas side must be maintained at a level higher than the pressure of oxygen gas side but less than the minimum pressure at which the blood is expelled through the membrane.

If the critical surface tension of the membrane is greater than the surface tension of blood, the membrane will be fully wetted with blood and the micropores therein will be filled with blood. Since the principle of microporous membrane oxygenators is to diffuse oxygen gas and carbon dioxide gas in opposite directions in a gaseous phase defined by each of the microholes of membrane, the oxygenator cannot function properly if such microholes are filled with blood. This could be avoided by applying a higher pressure to the oxygen gas side than to blood side but this approach will give rise to a serious danger of introducing oxygen gas bubbles into the blood and returning the same to the patient. It is for this reason that the pressure of blood side should be higher than the pressure of oxygen gas side and the critical surface tension should be less than the surface tension of blood at 37°C. For example, the surface tension of standard bovine whole blood is found to be about 65 dyne/cm. The surface tension of human blood varies depending upon health conditions and the body temperature of a particular patient but generally ranges from about 60 to about 70 dyne/cm. Accordingly, the membrane should have a critical surface tension below said value.

If the pressure differential between the blood side and oxygen gas side exceeds a certain limit, the blood will be forced to fill microholes in the membrane. This pressure differential at which the blood begins to fill the microholes is hereinafter referred to as "minimum penetration pressure". This value varies with the material of particular membranes, pore size and configuration of microholes. For instance, the above-described membrane made of dichlorodiphenylsulfone-bisphenol condensate resin has a critical surface tension of about 50 dyne/cm at 37°C and the minimum penetration pressure of 220 kPa. Accordingly, if the pressure of blood side in this case is maintained at a level higher than the pressure of oxygen gas side keeping the pressure differential within said value, blood will not pass to the oxygen gas side or alternatively oxygen gas will not pass to the blood side through the membrane.

The membrane used in the oxygenator of this invention is an anisotropic membrane having a skin layer including a number of microholes. The pore size of microholes is large enough such that the flow of gas therethrough is permitted as Knudsen flow rather than the dissoluton-diffusion. To this end the average pore size ranges from about 1 nm to 10 nm, preferably from about 2 nm to 5 nm. To achieve the desired high permeability the skin layer has a thickness from 0.01 to 3 μm but typically about 1 μm is preferable for strength reason. The pore size and thickness may be easily determined by an electron microscope.

The porous layer has a pore size from 0.1 to 20 μm, and a thickness from 10 to 500 μm, particularly from 20 to 70 μm. The pore size and thickness of porous layer may also be determined by an electron microscope.

The porosity of the membrane is desirably as great as possible unless the strength property of membrane is unduly impaired.

A pair of membranes may be used as a single unit with skin layers facing each other. Alternatively, a single membrane may be overlaid by a suitable support such as polypropylene nonwoven fabrics, porous sheets or nettings.

The material of the membrane used in the present invention has a contact angle with blood less than 90°. If a mateial having a contact angle higher than 90°, for example, polytetrafluoroethylene is used, oxygen gas bubbles may be generated in the blood when the pressure of oxygen gas exceeds the pressure of blood side. Therefore, the pressure differential across the membrane must be controlled accurately and a precise safety device is required. With the use of a material having a contact angle less than 90°, however, generation of oxygen gas bubbles may be avoided provided that the reversal of pressure differential is kept within a certain limit. This limit varies depending upon the maximum pore size and configuration of pores on blood side. When, for example, the abovementioned dichlorodiphenylsulfone-bisphenol resin membrane is used with the skin layer facing the blood side, the value of this limit is from 6,66 kPa to 20 kPa. This eliminates the danger of returning oxygen gas bubbles to the patient due to accidental reversal of pressure differential between the blood side and the oxygen gas side.

As previously stated, the critial surface tension of the membrane should be less than the surface tension of blood at 37°C and specifically less than 70 dyne/cm, preferably less than 60 dyne/cm.

Any polysulfone material having the above described parameters may be used. Polysulfone resins are of excellent bio-compatibility. A polyether sulfone resin described in Chemtech. July 1975, pages 426 - 432 such as those sold under commercial names of Astrel[T] 360(3M), polyether sulfone 720P and 200P (I.C.I.) and Udel Polysulfone[T] (U.C.C.) may be used. The membrane use in the present invention may be prepared from these materials by any known method. For example, a method described in Japanese Laid Open Patent Application 42 765/1976 or 16 381/1979 may be employed for the manufacture of the membrane used in the present invention.

The oxygenator of this invention may be of any known design using the membrane in various forms such as envelopes, flat sheets, hollow fibers and the like. For example, an elongated envelope of membrane may be wound around a cylindrical core to form a coil and placed in a casing. Inlet and outlet ports are provided in communication with each flow path defined in the interior and the exterior of the envelope. An oxygenator of this design is shown in Japanese Laid Open Patent Application 135 488/1974 (equivalent to FR-A-2 206 954).

Alternatively, a plurality of envelopes of membrane may be stacked with periphery of each open end being bonded together, and placed in a casing. Manifold means are provided adjacent opposite ends and opposite sides of the stack to communicate with their respective ports. An example of this design may be seen in a dialyzer described in Japanese Patent Publication 22 960/1973.

In these oxygenators, the blood path may be either interior or exterior of the envelope but preferably faces the skin layer of membrane. It is also desirable that each turn or wall of the envelope is spaced from the adjacent ones by a spacer or disturber to permit fluid flow and to produce a turbulent flow for reducing the boundary film resistance. Suitable spacer or disturber means are a mesh screen made of inert material.

Another design of oxygenators may be seen in Japanese Laid Open Patent Application 12 873/1978. Here, the membrane is convoluted to define a pair of plurality of fluid paths in interleaving relation on opposite sides of membrane. Each one of the pair of fluid paths communicates with an inlet and an outlet. It is also desirable for this design to place screen support means between the adjacent turns of membrane.

In these stacked or convoluted membrane oxygenators, it will be understood that a plurality of anisotropic membrane walls are overlaid in face-to-face and back-to-back relations alternately and a pair of plurality of fluid flow paths are formed on opposite sides of anisotropic membrane.

The oxygenator of this invention may be of hollow fiber design. In this case a bundle of a large number of hollow filaments is placed in a cylindrical casing and held in position within the casing by bonding filaments together and with the inside wall of the casing at both ends with a curable potting compound. This construction is popular in hollow fiber hemodialyzers.

This invention will be further described by way of example by referring to the accompanying drawings in which

Figure 1 shows a section in the vicinity of skin layer viewed by an electron microscope;
Figure 2 shows an experimental oxygenator;
Figure 3 shows a circuit for carrying out a hemolysis test;
Figure 4 shows a perspective view of a further embodiment of a blood oxygenator; and
Figure 5 shows a cross-sectional view of a blood oxygenator according to Figure 4.

To further illustrate the invention, the following non-limiting examples are presented. Unless otherwise indicated all percents are by weight.

## Example 1

A solution consisting of:

| | |
|---|---|
| polysulfone (UDEL polysulfone P1700.U.C.C.) | 22 % |
| propylene glycol | 14 % |
| and, N-methyl-2-pyrrolidone | 64 % |

was cast on a polyester film uniformly and soaked in water at 30°C. The resulting membrane became strippable from the polyester film after complete coagulation. This membrane was withdrawn, thoroughly washed with water and dried at 140°C for 2 minutes.

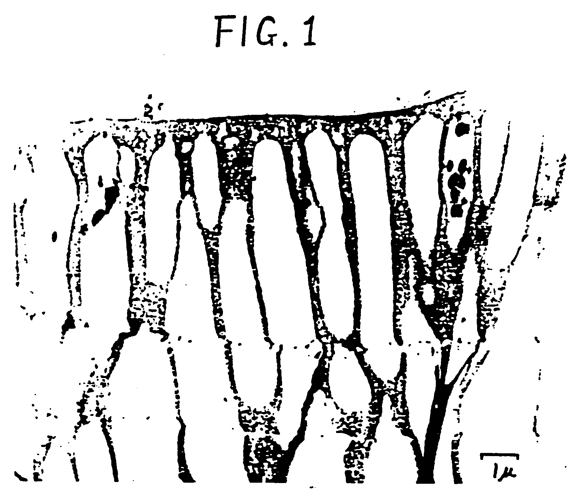

The membrane has a thickness of 60 μm, a moisture content of 1.2 % and a yield point of 390 g/cm at a test speed of 1 cm/sec. A section in the vicinity of the skin layer of this membrane viewed by an electron microscope is shown in Figure 1.

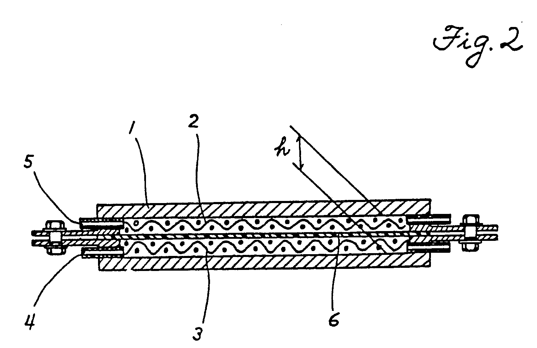

An experimental oxygenator shown in Figure 2 was assembled using this membrane as membrane 6 in Figure 2. The width h of gas exchange chamber of gas exchanger 1 is 0.8 mm. Screens 2 and 3 are placed in flow paths on opposite sides of membrane 6 and each are polyester netting having 118 strands/m of 100 x 50 mm size.

Water was pumped through the inlet port 4 at a flow rate of 430 ml/min, while oxygen gas was flown through the inlet port 5 at a flow rate of 1,000 ml/min. The amount of absorbed oxygen was determined from partial pressures of oxygen Pi and Po at inlet and outlet, and the mass transfer coefficient relative to oxygen was calculated by the following equation:

$$\frac{dV}{dt} = KA\alpha \frac{P_G - P_L}{760} \times \frac{760}{714} \times \frac{310}{273}$$

wherein V is the volume of absorbed oxygen ($cm^3$). A is the effective surface area of membrane ($cm^2$), $\alpha$ is the solubility constant of oxygen in water ($cm^3 O_2/cm^3 H_2O$ atm.), $P_G$ is he partial pressure of oxygen in gas phase, and $P_L$ is the partial pressure of oxygen in water.
The results obtained are as follows:

When flowing water on the skin layer side,

$$K = 110 \times 10^{-4} \text{ cm/sec.,}$$

and when flowing oxygen gas on the skin layer side,

$$K = 55 \times 10^{-4} \text{ cm/sec.}$$

In a similar way, the mass transfer coefficient relative to carbon dioxide was determined. The results obtained are as follows:
When flowing water on the skin layer side,

$$K = 102 \times 10^{-4} \text{ cm/sec.,}$$

and when flowing carbon dioxide gas on the skin layer side,

$$K = 43 \times 10^{-4} \text{ cm/sec.}$$

A pressure differential was created across the membrane by elevating the gas pressure and the value at which bubbles begin to appear in the water phase (bubbling point) was determined. The bubbling point was 20 kPa when flowing water on the skin layer side, and 17 kPa when flowing gas on the skin layer side, respectively.
Various parameters of the anisotropic membrane used in the example are as follows:
Skin layer; about 0.5 μm thick, pore size less than 0,01 μm.
Porous layer; about 60 μm thick, pore size 0.1 - 7 μm.
Critical surface tension; 50 dyne/cm.
Minimum penetration pressure; 220 kPa at 37°C.
Contact angle (blood); 57° at 37°C.

## Example 2

A solution consisting of:

| | |
|---|---|
| polysulfone (UDEL polysulfone P 1700, U.C.C.) | 23 % |
| propylene glycol | 16 % |
| and. N-methyl-2-pyrrolidone | 61 % |

was extruded through an annular nozzle while filling the interior of extrudate with water. The extrudate was fully coagulated in a water bath, thoroughly washed with water and dried at 120°C for 10 minutes.

The resultant hollow filament has an inner diameter of 0.2 mm, an Outer diameter of 0.3 mm and a wall thickness of 0.05 mm. This hollow filament has skin layers on both inner and outer surfaces each having a thickness of about 0.2 μm and minute pores of a pore size less than 0,01 μm. The porous layer has a thickness of about 50 μm and a pore size of 0.1 - 3 μm.

The minimum penetration pressure of this hollow filament was determined by filling the interior of hollow filament with blood. The value obtained was 210 kPa at 37°C. The critical surface tension of this hollow fiber membrane was 50 dyne/cm at 37°C and the contact angle with blood at 37°C was 53°.

An oxygenator was assembled from this filament. 3060 filaments of an effective length of 18 cm were bundled to give an effective inner surface area of 3700 cm$^2$. Using the resultant oxygenator water was pumped through the interior of the hollow filaments at a flow rate of 320 ml/min, while oxygen gas was flown along the exterior of filaments at 1000 ml/min. The mass transfer coefficient measured as in Example 1 was $32 \times 10^{-4}$ cm/sec., and the bubbling point was 19,33 kPa.

Similarly, the mass transfer coefficient relative to carbon dioxide was determined and found to be $34 \times 10^{-4}$ cm/sec.

The ratio of mass transfer coefficient of oxygen gas to that of carbon dioxide gas is 0.94, which is far lower than the corresponding ratio of a commercial silicone membrane oxygenator, which is about 2. This means that the membrane used herein is highly permeable to carbon dioxide gas as well.

This oxygenator was then dried by passing dry air at room temperature through the interior of hollow filaments at a flow rate of 3 liter/min for 5 hours. Bovine whole blood having a hematocrit value of 35 % was pumped thrugh the hollow part of the filaments at a flow rate of 380 ml/min at 37°C, while oxygen gas was flown along the exterior of the filaments at 1000 ml/min. The outlet of oxygen gas was opened to the atmosphere and, therefore, the gauge pressure of oxygen gas within the oxygenator was nearly zero. The pressure of the blood side within the oxygenator was 21.33 kPa at the blood inlet, and 4,1 kPa at the blood outlet, respectively. The partial pressure of oxygen in the blood was 5,453 kPa at the inlet and 107 mmHg at the outlet, respectively.

The partial pressure of carbon dioxide in the blood was 4.8 kPa at the inlet and 3,97 kPa at the outlet, respectively. The oxygen gas uptake was then 20.0 ml/min.

These data show that the performance of the oxygenator of this example may be accepted in practical use.

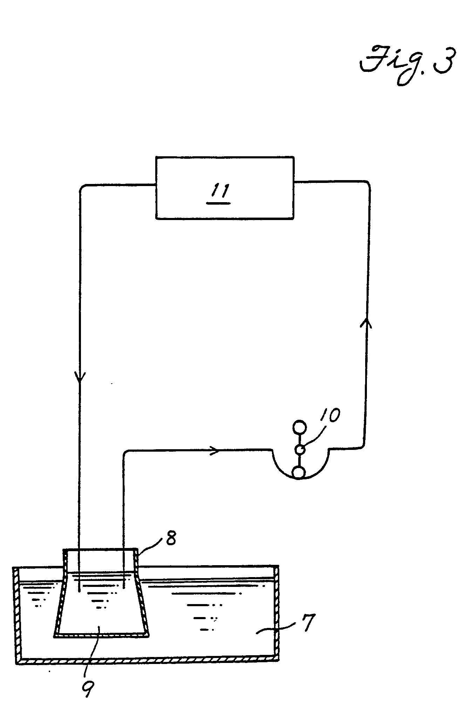

A hemolysis test was carried out with the above oxygenator using a circuit schematically shown in Fig. 3. 1,000 ml of heparine added bovine whole blood 9 having a hematocrit value of 35 % was placed in a reservoir 8 and maintained at a constant temperature of 37°C in a water bath 7. Blood was circulated by a roller pump 10 through the oxygenator 11 at a flow rate of 380 ml/min. Oxygen gas was not supplied during the test. The amount of hemolysis per hour was 6.9 mg/dl · hour. For comparison, a similar test was carried out with a commercial Kolobow membrane oxygenator having an effective membrane surface area of 0.8 m$^2$. The amount of hemolysis in this case was 44 mg/dl · hour at a blood flow rate of 800 ml/min. This shows that the amount of hemolysis of the oxygenator of this example is very small.

## Example 3

Example 1 was repeated to give an anisotropic membrane of 100 μm thick, 300 mm wide and 10 m long. The resultant membrane has a skin layer of 0.5 μm thick having minute pores of a pore size less than 0.01 μm. The porous layer has a thickness of about 100 μm and a pore size of 0.1 - 7 μm.

The critical surface tension of this membrane was 50 dyne/cm at 37°C and the minimum penetration pressure was 230 kPa at 37°C. The contact angle with blood at 37°C was 57°.

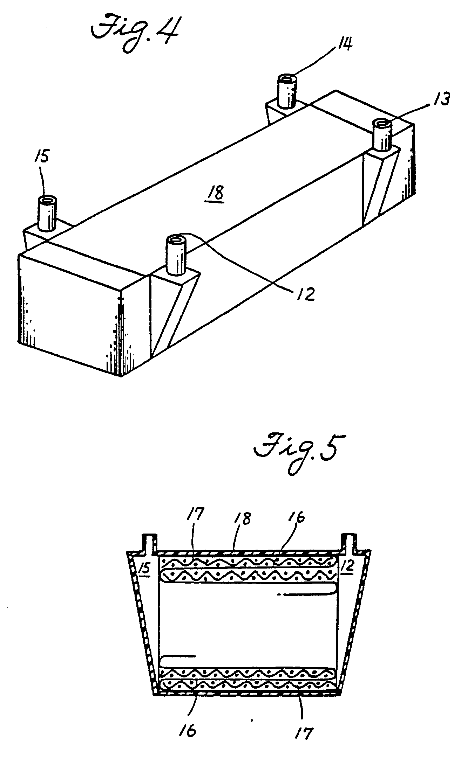

An oxygenator shown in Figure 4 and Figure 5 was assembled by convoluting the membrane 16 to define a pair of plurality of flow paths on opposite sides of membrane 16. Each turn of convoluted membrane 16 is spaced from the adjacent turns by a polyester mesh screen 17 of 30 μm thick and 300 x 50 mm in size. The resulting rectangular assembly having a size of 300 x 55 x 65 mm was placed in a casing shown in Figure 4 and sealed to inner walls of casing with an adhesive except those areas facing port chambers 12 through 15. The effective surface area of the resultant oxygenator was about 1.6 m$^2$.

Water was pumped through flow paths on the skin layer side of the mebrane at a flow rate of 1,600 ml/min, while oxygen gas was passed through flow paths on the porous layer side of membrane at a flow rate of 1,500 ml/min.

The mass transfer coefficients relative to oxygen and carbondioxide, measured as in Examples 1 and 2, were $37 \times 10^{-4}$ cm/sec and $40 \times 10^{-4}$ cm/sec, respectively. The bubbling point was 14,93 kPa.

As in Example 2, the oxygenator was dried, and bovine whole blood having a hematocrit value of 35 % was passed through flow paths on the skin layer side at a flow rate of 1,600 ml/min at 37°C, while oxygen gas was passed through flow paths on the porous layer side at a flow rate of 1,500 ml/min. The gauge pressure of oxygen gas was nearly zero. The pressure of the blood side was 26,397 kPa at the inlet and 5,2 kPa at the outlet, respectively. The partial pressure of oxygen in blood was 4,68 kPa at the inlet and 23,465 kPa at the outlet, respectively. The partial pressure of carbon dioxide in blood was 4,8 kPa at the inlet and 3,9 kPa at the outlet, respectively. The oxygen uptake was 113.1 ml/min.

Hemolysis test was carried out on this oxygenator as in Example 2. The amount of hemolysis was 8.3 mg/dl hour.

The above has been offered for illustrative purposes only, and it is not for the purpose of limiting the scope of this invention which is defined in the claims below.

## Claims

A blood oxygenator for oxygenating blood and simultaneously removing carbon dioxide therefrom comprising a semi-permeable non-hydrophilic membrane (6, 16) and a first flow path and a second flow path positioned on opposite sides or said membrane (6, 16) in gas exchange relation, whereby the membrane (6, 16)
- is made or a polysulfone resin,
- has a critical surface tension less than the surface tension of human blood at 37° C,
- comprises a porous layer having a thickness of 10 to 500 μm and a pore size of 0.1 to 20 μm and integral with the porous layer a skin layer of 0.01 to 3 μm thickness, having minute pores of a pore size of 1 nm to 10 nm.

2. The blood oxygenator of claim 1, wherein the form of said membrane (6, 16) is a flat sheet.

3. The blood oxygenator of claim 1, wherein the form of said membrane (6, 16) is an envelope.

4. The blood oxygenator of claim 1, wherein the form of said membrane (6, 16) is a hollow filament.

5. The blood oxygenator of claim 1 or 2, wherein two sheets of said membrane (6, 16) are combined to form a single unit with said skin layer facing each other.

6. The blood oxygenator of claim 1 or 2, wherein said membrane (6, 16) overlies an associated porous support.

7. The blood oxygenator of claim 1, wherein one of said flow paths is disposed on said skin layer side and adapted to pass blood therethrough, and the other flow path is disposed on said porous layer side and adapted to pass oxygen gas therethrough.

8. The blood oxygenator of claim 7, wherein said skin layer is dry.

## Patentansprüche

1. Blut-Oxygeniergerät,um Blut zu oxygenieren und gleichzeitig Kohlendioxid aus ihm zu entfernen, mit einer semipermeablen nichthydrophilen Membran (6, 16), einem ersten und einem zweiten Strömungspfad, die auf einander gegenüberliegenden Seiten der Membran (6, 16) so angeordnet sind, daß sie Gas austauschen können, wobei die Membran (6, 16)
- aus einem Polysulfonharz gefertigt ist,
- eine kritische Oberflächenspannung aufweist, die unterhalb der Oberflächenspannung des menschlichen Blutes bei 37° liegt,
- und eine poröse Schicht mit einer Dicke zwischen 10 und 500 μ m und einer Porengröße zwischen 0,1 und 20 μm sowie eine mit der porösen Schicht einstückige Deckschicht mit einer Dicke zwischen 0,01 und 3 μ m, die kleine Poren aufweist mit einer Größe zwischen 1 und 10 nm, umfaßt.

2. Blut-Oxygeniergerät nach Anspruch 1, wobei die Membran (6, 16) die Gestalt eines flachen Blattes hat.

3. Blut-Oxygeniergerät nach Anspruch 1, wobei die Membran (6, 16) die Gestalt einer Umhüllung hat.

4. Blut-Oxygeniergerät nach Anspruch 1, wobei die Membran (6, 16) die Form einer Hohlfaser hat.

5. Blut-Oxygeniergerät nach Anspruch 1 oder 2, wobei zwei Blätter der Membran (6, 16) unter Bildung einer einzelnen Einheit zusammengefaßt sind, bei der die Deckschichten einander gegenüber liegen.

6. Blut-Oxygeniergerät nach Anspruch 1 oder 2, wobei die Membran (6, 16) über einem zugeordneten porösen Träger liegt.

7. Blut-Oxygeniergerät nach Anspruch 1, wobei einer der Strömungspfade an der Deckschichtseite angeordnet und zum Durchleiten von Blut geeignet ist, der andere Strömungspfad auf der Seite der porösen Schicht angeordnet ist und zum Durchleiten von Sauerstoffgas geeignet ist.

8. Blut-Oxygeniergerät nach Anspruch 7, wobei die Deckschicht trocken ist.

## Revendications

1. Oxygénateur de sang, pour oxygéner le sang et en éliminer simultanément le gaz carbonique, comprenant une membrane semi-perméable non hydrophile (6, 16), un premier passage de circulation et un deuxième passage de circulation disposés de part et d'autre de ladite membrane (6, 16) en relation d'échange de gaz, dans lequel la membrane (6, 16)
- est fabriquée en une résine de type polysulfone,
- possède une tension de surface critique inférieure à la tension de surface du sang humain à 37°C,
- comprend une couche poreuse ayant une épaisseur de 10 à 500 μm et une dimension de pore de 0,1 à 20 μm et, solidaire de la couche poreuse, une couche de peau de 0,01 à 3 μm d'épaisseur à micropores d'une dimension de pore de 1 nm à 10 nm.

2. Oxygénateur de sang suivant la revendication 1, dans lequel la forme de ladite membrane (6, 16) est une feuille plane.

3. Oxygénateur de sang suivant la revendication 1, dans lequel la forme de ladite membrane (6, 16) est une enveloppe.

4. Oxygénateur de sang suivant la revendication 1, dans lequel la forme de ladite membrane (6, 16) est un filament creux.

5. Oxygénateur de sang suivant la revendication 1 ou 2, dans lequel deux feuilles de ladite membrane (6, 16) sont combinées pour constituer une seule unité, lesdites couches de peau étant en regard l'une de l'autre.

6. Oxygénateur de sang suivant la revendication 1 ou 2, dans lequel ladite membrane (6, 16) recouvre un support poreux associé.

7. Oxygénateur de sang suivant la revendication 1, dans lequel l'un desdits passages de circulation est disposé du côté de ladite couche de peau et est prévu pour la circulation de sang, et l'autre passage de circulation est disposé du côté de ladite couche poreuse et est prévu pour la circulation d'oxygène gazeux.

8. Oxygénateur de sang suivant la revendication 7, dans lequel ladite couche de peau est sèche.

EP 0 041 692 B2

# FIG. 1

*Fig. 2*

*Fig. 3*

*Fig.4*

*Fig.5*